# EUROPEAN PATENT APPLICATION

(11) **EP 1 072 274 A1**
(43) Date of publication of application: **31.01.2001**
(21) Application number: 00306469.8
(22) Date of filing: 28.07.2000
(51) Int. Cl.: A61L 2/08, A61L 27/16

(54) **Two step gamma irradiation of polymeric bioimplant**

(30) Priority: 29.07.1999 US 363591
(71) Applicant: DEPUY ORTHOPAEDICS, INC., Warsaw, Indiana 46581 (US)
(72) Inventor: Hamilton, John, V., Foxborough, Massachusetts 02035 (US); Schmidt, Mary, Beth, Pomfret Centre, Connecticut 06259 (US); Greer, Keith, South Easton, Massachusetts 02375 (US)
(74) Representative: Belcher, Simon James

(57) **Abstract**

Wear resistance and oxidation resistance of a bioimplantable polymeric part is improved by packaging a bulk or unfinished polymeric object within gas impermeable package or container substantially free of oxygen, irradiating the packaged object with penetrating radiation to an extent sufficient to initiate or effect a desired substantial level of cross-linking within the polymer, and heating the package object to a point above the polymer melt temperature effective to stabilize the material against subsequent oxidative change. The stabilized material or semi-finished parts are then machined or otherwise formed into finished parts, repackaged and sterilized. Sterilization may be effected by re-packaging in vacuum foil and irradiating with an additional dose of gamma radiation. The procedure achieves very high levels of cross linking while limiting the radiation damage that is generally associated with high dose radiation, reducing susceptibility to aging or chemical degradation and avoiding the risks involved in post-treatments with reactive materials.

## Description

### REFERENCE TO RELATED APPLICATIONS

This application is related to the United States patent application entitled **LOW TEMPERATURE PRESSURE STABILIZATION OF IMPLANT COMPONENT** (Attorney Docke No. 22675-176) and the United States patent application entitled **TWO STEP GAMMA IRRADIATION OF POLYMERIC BIOIMPLANT** (Attorney Docket No. 22675-177) each filed simultaneously herewith on July 29, 1999 by Express Mail with Express Mail Label No. EJ000510233US and No. EJ0005510247US, respectively, by the present inventors. Each of the foregoing patent applications is hereby incorporated herein by reference in its entirety.

### BACKGROUND OF THE INVENTION

This invention relates to bioimplantable polymeric articles and more particularly to materials treatment methods for improving the wear resistance and oxidation resistance of such articles.

Advances in biomedical engineering have resulted in numerous polymeric articles to be implanted within the body. Polymeric components are widely used in orthopedic surgery, for example, to form articulation surfaces within artificial joints. Ultrahigh molecular weight polyethylene (UHMWPE) is an example of a polymer that is commonly used to form components of artificial joints.

Among the properties required of bioimplantable polymeric components, particularly those used in artificial joints, are low friction, biocompatibility, and good mechanical properties, including excellent resistance to wear. Such components must also be sterile before implantation within a patient. by forming the component to a very exacting contour and surface finish, utilizing a material that has good stiffness, strength, hardness, toughness and resistance to creep. It must also have suitable physical properties to resist various common forms of material failure or degradation.

Some polymers and medical devices may be adversely affected by heat sterilization, so this technique is not widely used. Ethylene oxide sterilization, commonly used in other contexts for sterilizing medical devices, can pose health and environmental risks that also render this method less desirable. As a result, a preferred method of sterilizing many medical articles, including polymeric components, is by exposure to forms of ionizing radiation such as gamma ray, x-ray, or electron beam radiation.

Presently, sterilization by gamma radiation is a preferred method for sterilizing many medical devices, and is preferred by applicant for cross-linking as well as sterilizing bioimplantable polymeric components. One potential effect of gamma irradiation is that the gamma rays can initiate chemical reactions within the polymer that can affect the structure, morphology and some mechanical properties of the polymer. During gamma irradiation a variety of chemical species, such as ions, excited molecules, double bonds, oxidation products and free radicals are created within the polymer. Free radicals are believed to be a species generated during gamma irradiation that may contribute most to changes in the properties of the irradiated polymers.

Once free radicals are formed within a polymer, these species may participate in at least four major types of reactions. The free radicals can undergo a recombination reaction by reacting with hydrogen to eliminate the free radical, by reacting with carbon molecules to create side chains, or both. Free radicals can also initiate or participate in a chain scission reaction that results in a decrease in the molecular weight, and an increase or change in the density and crystallinity, of the polymer, thus causing some mechanical properties of the polymer to degrade. Cross-linking is another reaction in which the free radicals can participate. Finally, the free radicals may remain trapped within a polymeric material for an extended time period (e.g., years) without initially reacting, but remaining available to react as conditions dictate.

The presence of oxygen in the polymeric material or in its surrounding environment can contribute to an oxidation reaction in which free radicals and dissolved oxygen react to produce a compound with a carbonyl functional group, which then initiates chain scission and results in the creation of new free radicals. Thus, oxidation can decrease the molecular weight of a polymer (due to chain scission), which may contribute to the degradation of its mechanical properties.

Gamma irradiation of polymer components in air, while cross-linking and thus enhancing wear properties, is believed to bring about a gradual decrease in the wear resistance of the polymer due, in part, to such oxidation effects. Because wear resistance is a key mechanical property for polymeric components that are used in joint prostheses, several approaches have been developed to address this effect. One current practice addresses this problem by irradiating polymeric components in an environment of an inert gas (e.g., argon, helium, nitrogen) to minimize oxidation effects. *See*, Kurth, M. et al., "*Effects of Radiation Sterilization on UHMW-Polyethylene*" Antec 87, pp. 1193-1197 (1987); Streicher, R.K., *Radiol. Phys. Chem.,* Vol. 31, Nos. 4-6, pp. 693-698 (1988); Streicher, R.M., "Improving UHMWPE by Ionizing Radiation Cross-linking During Sterilization", 17th Annual Meeting of the Society for BioMaterials, p. 181 (1991). Others have used vacuum techniques to help purge an environment of oxygen before conducting gamma radiation sterilization. *See*, Yong Zhao, et al., *J. Appl*. *Polymer Sci*., Vol. 50, pp. 1797-1801 (1993). In addition to utilizing ionizing radiation to effect sterilization and taking steps to deal with potentially undesirable side effects, various researchers or commercial prosthesis manufacturers have employed large dose irradiation specifically to cross-link or possibly toughen the prosthesis material so as to increase wear resistance. Generally the doses effective to introduce a substantial level of cross-linking may be higher than is needed for sterilization, and the concentration of trapped free radicals will be greater. in a sliding joint surface wetted with biological and undergoing repetitve movement, while a property of great importance to artificial joint components, may be hard to accurately predict. Natural friction and aging processes within a replaced artificial joint can cause minute particles of debris (e.g., particles from a polymeric component) to become dislodged and to migrate within the joint. This phenomenon of wear debris within artificial joints is a serious problem that can inhibit the proper mechanical functioning of the joint and also lead to osteolysis and bone deterioration. These biological reactions may depend strongly not only on the amount of debris, but on the particle size. If osteolysis develops around an artificial joint, correction typically requires surgical removal of the diseased tissue and revision of the artificial joint. Thus, in the context of prosthetic joints, "wear" is a complex phenomenon. Its degree can be estimated based on measurement of a joint wear simulated with robotic testing machines capable of repetitive motion once the material properties are optimized.

Wear resistance depends on factors such as the hardness and toughness of the polymer material, which may in turn depend upon specific properties such as the molecular weight of the resins, the degree of cross linking of the material, and the size, shape, distribution or relative amounts of amorphous and of crystalline polymer. Each of these basic properties may be altered by the application of radiation, heat or chemical agents. Moreover, the fabrication of polymeric components generally proceeds from a solid or resin starting material, which is provided as either a powder or granular resin material, or as a consolidated blank, e.g., sheet, rod, puck or block or preform, which must be machined to final form. Heating involved in initial resin consolidation or shaping steps may influence underlying physical properties, and cross-linking irradiation may be used in forming the finished wear-resistant solid article. Different forms of irradiation may each have different absorption or interaction characteristics, or generate heat which affects other properties. Thus, wear resistance depends in a rather complex way upon a number of processing steps which may be employed to physically form, treat or finish the polymer component.

Because wear resistance is a property of such importance for polymeric components used to form artificial joints, it would be advantageous to be able to provide a method for increasing the wear resistance of bioimplantable polymeric components.

It is thus an object of the invention to provide methods for cross-linking and sterilizing polymer components which impart improved wear resistance.

It is also an object to provide a cross-linking technique for medical grade implantable polymer components that impart or preserve important properties of the components.

A further object is to provide a bioimplantable polymeric component having a high degree of cross-linking and being less prone to the effects of oxidation.

These and other objects will be apparent to one of ordinary skill in the art upon reading the description that follows.

### SUMMARY OF THE INVENTION

One or more of these and other desirable properties are achieved in a method of the invention for increasing the wear resistance of polymeric parts. The method is particularly well suited to treat articles formed of a biocompatible polymer and intended for use as components of an artificial joint. A variety of polymeric materials, particularly olefins such as ultra high molecular weight polyethylene (UHMWPE) material, can be treated according to the method of the invention to improve wear resistance and to improve oxidation resistance.

According to the method of the invention, the bulk polymeric material or unfinished component for forming a manufactured finished polymeric part, such as a prosthetic joint wear surface component, is placed within a gas impermeable, sealable package or container. The package and the material therein are then subjected to removal of surrounding oxygen by a vacuum or partial vacuum and/or backfilling, and are sealed. Next, the package and the material contained within the package is a first or target level of cross-linking of the polymer that forms the part. Various forms of ionizing energy can be used to treat the material. However, the use of gamma radiation with an energy above 0.5 MeV is the preferred radiation technique.

While the irradiated bulk material or unfinished part is still in the packaging, it is heated in the absence of oxygen above its melt point for a time effective to substantially eliminate the free radicals created by the irradiation cross linking step, and thus to stabilize the treated material. It is then slowly cooled, e.g., at about 5°C/hr. The material or parts so treated are then removed from the package, and may be machined or otherwise worked to form finished components. The finished components are next repackaged in vacuum barrier pouches and subjected to a dose of penetrating radiation at a lower level effective to sterilize the components without impairing their physical properties. In particular, the second radiation dose is kept to a level that, while introducing additional cross-linking, does not reintroduce a substantial or deleterious level of free radicals.

This two-step irradiation process with intermediate stabilization has been found to be an effective process to achieve a high level of cross-linking for the underlying material effective to increase the wear resistance of polymeric parts without impairing or degrading other mechanical properties that are important to such parts. An additional benefit of the invention is the improved ability of the polymeric parts to resist oxidation. The resulting sterilized part is characterized by a substantially reduced oxidation index extending with a depth profile to a millimeter or more at the exposed surface of the article to provide sustained wearability in use.

### BRIEF DESCRIPTION OF THE DRAWINGS

Figure 1 is a graph which plots oxidation index for two samples evaluated in Example 1.
Figure 2 is a flow chart showing steps of a method in accordance with the present invention.

### DETAILED DESCRIPTION OF THE INVENTION

The present invention provides a method for improving the wear resistance of manufactured polymeric parts. According to this process the bulk material for a desired polymeric part is first consolidated (if a resin or powder), and/or is formed into a rough or unfinished part, by known processes, such as compression molding, ram extrusion, injection molding and possibly partial or rough machining. It is then irradiated to introduce a first degree of cross-linking. The polymer that forms the part can be low density polyethylene, high density polyethylene, ultrahigh molecular weight polyethylene (UHMWPE), polypropylene, polyester, nylon, polyurethane, poly(methylmethacrylate), or other biocompatible polymer typically used in biomedical applications. A preferred polymer is UHMWPE, which is commonly used in a variety of orthopedic implants, such as liners for acetabular shells of artificial hip joints, or tibial bearing members in artificial knee joints. The invention will now be described with reference to such a UHMWPE component.

In one embodiment, the bulk polymeric material or unfinished parts that are to be subjected to the process of the invention are first placed in an isolation compartment such as a flexible package that is formed of a gas-impermeable film assembly and is heat sealable. The bulk material may, for example, take the form of a sheet, puck, rod, bar, billet or other common shape resulting from or following the consolidation process utilized on a starting resin. As applied to an unfinished part, the unfinished part may, for example, be a compression molded oversize blank or a partially finished part machined from a previously consolidated blank or bulk material. This blank or part is to be subjected to precision shaping or finishing after carrying out the first step or steps of the invention. This starting part of material will henceforth be referred to, interchangeably, as either the polymer object or the prosthesis blank. More than one polymer object can be placed in a single package. The package and the object are then subjected to a vacuum while the heat sealable package remains open and the vacuum is maintained for a time effective to evacuate the bag and at least partially degas the object. Thereafter, the package is heat sealed while maintaining the vacuum, thus closing the gas impermeable package so it remains evacuated. Preferably a high embodiments the package may be back-filled with an inert gas. In each case the package interior is free of oxygen and other adversely reactive or reactable gases.

Thus, the packaging of the polymeric object in a heat sealed package under vacuum is carried out to remove oxygen from the polymeric material and from the environment within the package and preferably to reduce pressure within the package. The package preferably includes one or more barrier layers, such as metal foil, making it impermeable to gaseous hydrogen. This assures that hydrogen released during radiation treatment remains trapped in the package.

Following the vacuum heat sealing of the package that contains the polymeric object, the package and the object are irradiated with penetrating radiation in an amount that is sufficient to produce substantial cross-linking of polymer chains within the polymer material. Various acceptable forms of ionizing radiation can be used to effect the sterilization of the part forms including gamma rays, x-rays, and electron beam radiation. Gamma radiation is currently preferred for providing effective penetration of package wall as well as of the body of the polymer object, thus assuring a fairly uniform level of exposure and cross-linking in the polymer blank, allowing the flexible application of secondary machining steps without loss of surface strength, as discussed further below.

The compliant packaging material within which the polymeric parts are sealed can be selected from among many types of high barrier, flexible packaging material that are commonly used to enclose medical devices. Preferably the packaging material is a multilayered, heat seal, peelable packaging material that includes a barrier layer, various polymer layers and a heat seal coating. Examples of suitable materials are those that include the following layers: polyester film-low density polyethylene-foil-ionomer-heat seal coating. Packaging materials having the following layers can also be used: polyester-low density polyethylene-foil-EAA-linear low density polyethylene-heat seal coating; and polyester-Surlyn-nylon-Surlyn-foil-EAA-linear low density polyethylene-heat seal coating. Suitable packaging materials can be obtained from a variety of sources, including Tolas Health Care Packaging of Feasterville, Pennsylvania. The thickness of the packaging material preferably is in the range of about 2 mils to 7 mils (50-175 micrometers). Techniques for vacuum heat sealing such packaging material are readily available to those of ordinary skill in the art. Suitable vacuum packaging equipment for heat sealing packages under vacuum will be known to those of ordinary skill in the art. One suitable vacuum packaging apparatus is a MultiVac A342 unit, available from Multivac, Inc. of Kansas City, Missouri. Optionally, the package may be backfilled with an inert gas, such as nitrogen or a noble gas.

As noted above, the packaged polymeric material is irradiated using penetrating radiation, which is preferably gamma radiation from a source such as a Cobalt 60 source. The gamma radiation is administered for a duration and at a dosage level which is effective to introduce or initiate a substantial level of cross-linking. A dosage of approximately 40 kGy has been found to produce cross-linking at an acceptable level for one existing prior art prosthetic component; the present invention seeks to attain a level substantially higher, so a dose of approximately 60-90 kGy is preferred for UHMWPE resins commonly used in prosthesis manufacture. The irradiation dose may be delivered by exposure to gamma rays for several hours or longer, e.g., 10-14 hours.

In a variation of this first portion of the method of the present invention, the steps of packaging and irradiating may be implemented by placing the polymer object in anothrer type of container, either a rigid or a flexible container, such a a rigid sealed vessel. All or most of the oxygen is first evacuated from the container. Optionally, the container may next be pressurized with an inert gas (e.g., argon, helium or nitrogen) to approximately one-tenth to several atmospheres. Subsequently, the container and the enclosed polymeric parts are irradiated as described above to an extent sufficient to promote the desired substantial level of cross linking of the polymer. A variation of this embodiment dispenses with the need to evacuate the container by flushing it with a sufficient amount of an inert gas to displace all oxygen before irradiating the polymer object. free of oxygen, and irradiated with penetrating radiation to induce substantial cross-linking. A dose of 60 KGy has been found useful to cross link a GUR 1020 material to an effective level. By effective level, is meant a level measurably greater than the level typically achieved by a gamma irradiation dose of 40 kGy applied to prosthesis implant materials. As noted further below, certain features of the process result in further cross-linking during later stages of the process, so that, for example, by irradiating to achieve somewhat less cross linking than that which is ultimately desired, the final level can produce a very high level of cross-linking without corresponding increases in radiation damage. Thus, where the total desired cross-linking may require a dose of 80 kGy, the present invention may, for example, irradiate the packaged polymer object with a dose of 50-60 kGy at the first stage of processing.

Following irradiation, the polymer object is raised to a temperature above its melt point, e.g., to 135°C or more for a GUR 1020 resin UHMWPE material. This elevated heating promotes flexing and mobility of its long chain molecules, as well as enhanced diffusion of trapped or dissolved hydrogen, including the gases within the package or container that were released during irradiation of the polymer object. This allows isolated or immobilized free radicals to form cross-links or recombine, so that they are terminated and their activity is quenched. This thermal melting is effective to substantially reduce or eliminate the free radicals formed by the initial dose of radiation. The polymer is then slowly cooled from the melt temperature, e.g., at a rate of approximately 5°C/hour.

The foregoing steps of substantial cross-linking and thermal melting may introduce deformation or changes of dimension in the treated polymer, but result in a material or blank which is uniformly and highly cross-linked, and has been stabilized so that it does not contain a significant level of trapped free radicals and is not prone to degrade over time when subsequently stored or exposed to oxygenated environments.

In accordance with the method of the present invention, the treated blank or material so stabilized is then removed from the closed or sealed container, and is machined to form a finished prosthetic part, such as a liner or shell for an implantable prosthetic joint. For example, for making a liner for an acetabular cup, the material or unfinished component, after being irradiated and thermally treated as described above, may be machined to a final contour and finish.

Following machining, the finished part is then once again irradiated. In a preferred process, this is done by again vacuum packaging the part and then again treating it with a dose of penetrating radiation. A suitable dose may be obtained by exposure to gamma rays at a dose of 10-35 kGy which is effective to sterilize the component in the package. If desired, this second dose may be of a level that is high enough to introduce a further measurable amount of cross-linking. Preferably, however, the processing and finishing are arranged so that the component at this stage has a low bioburden, and the second radiation dose is set sufficiently low that, while effective to sterilize the component, it does not re-introduce a significant level of free radicals into the finished component, and the finished part is stable or may be readily stabilized after this step.

Thus, the treatment of the present invention involves irradiating the blank to introduce substantial cross linking, thermally elevating the irradiated blank above melt temperature in its processing container to stabilize the first radiation treatment, machining a finished article from the treated blank at this intermediate stage, and further irradiating the finished article. The material or blank is stabilized, curing long-term radiation damage in an intermediate step after substantial cross linking, allowing later steps to address sterility or to adjust the polymer mechanical properties substantially independently of the side effects generally associated with such high total dosed of radiation, and without degrading mechanical properties of the material. The resulting product has a very high degree of cross-linking but a low oxidation index.

The intermediate melt stabilization technique in a closed container of the present invention terminates reactive sites in the polymer and reduces the net amount of hydrogen that diffuses from or is lost by the bulk polymer, thus maintaining more hydrogen within the polymer. The conditions allow a relatively simple set of handling and treatment conditions to effectively minimize the extent of chain scission reactions number of free radicals within the polymer available to participate in oxidation reactions by recombining with the free radicals. The above-melt stabilization is also believed to contribute to additional cross-linking within the polymer, which is believed to increase the wear resistance of the polymer by increasing the molecular weight as well as reducing the potential of chain scission.

The following example serves to further illustrate the invention.

### EXAMPLE 1

Polymer objects were formed of a GUR 1020 resin and were sealed in flexible vacuum foil packages. A first set was irradiated with 80 kGy of gamma radiation, a level at which radiation damage is believed to have significant long-term effects on the material stability. A second set of polymer objects was irradiated with a relatively high dose of 60 kGy, followed by a thermal treatment in which the temperature was raised above the melt point (e.g. 150°C) and the objects were held at this temperature until completely melted. They were then cooled at a controlled rate of about 5°C/hour. The second group of polymer objects were then removed from their containers, machined into finished components, and repackaged in flexible vacuum foil bags. The finished components so packaged were then sterilized by applying a second, relatively low gamma radiation dose of about 20 kGy.

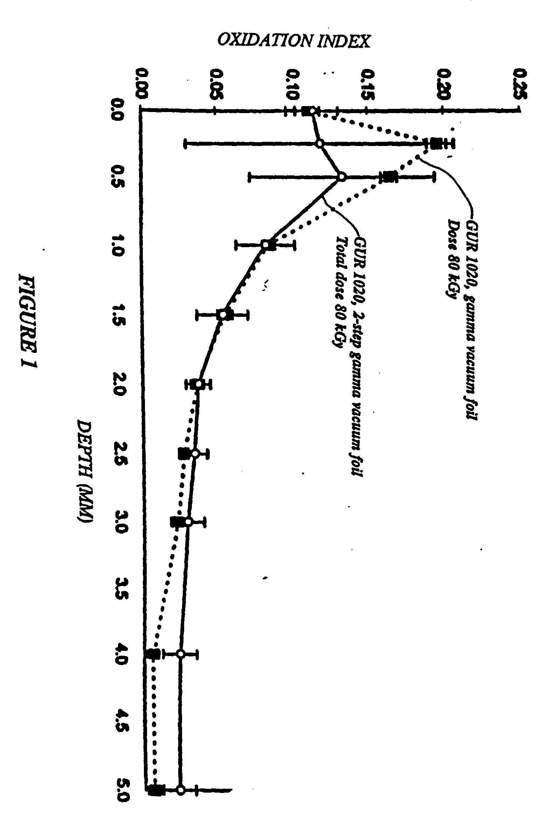

Figure 1 illustrates the oxidation index of the two sets of polymer objects, as a function of depth from the surface, determined after an oxidative challenge. As shown, the profile for the first set of objects rises to 0.2 in the outer layer within one millimeter of the surface, while the two step processing of the present invention limited the oxidation index to only slightly more than half that amount. By employing an intermediate melt-stabilization step, the stabilization process allows the immobilized radials to cross link or be terminated, while the subsequent machining avoids any problems of dimensional change that may have arisen during the melting process. A relatively low second radiation treatment at 20 kGy is adequate to achieve sterilization, but is sufficiently low to minimize free radical formation. The second dose also provides additional cross-linking, so the two step procedure achieves a cross-linking level at least as high as that of the single stage 80 kGy treatment of the first set, while maintaining an acceptably low oxidation index and avoiding dimensional problems.

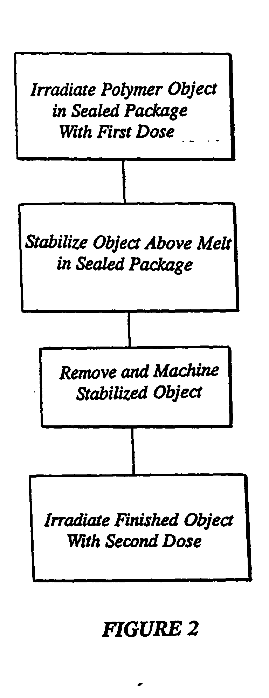

Figure 2 illustrates the method of this invention. As shown the bulk or unfinished polymer object is first irradiated to cross-link it to a relatively high level, and is stabilized while sealed off from oxygen and at a temperature above its melting point to produce a highly stable cross-linked intermediate object. This object is machined, and then again irradiated in a sealed container which may, for example, be its ultimate packaging. The second dose of radiation, while less than the first, introduces further cross-linking, but it is preferably sufficiently low that it does not introduce a deleterious level of radiation damage, such as excessive levels of trapped reactive species. The method thus allows one to achieve a high degree of cross-linking while avoiding dimensional problems and minimizing the risk that free radicals will result in long-term oxidative degradation of the prosthesis component.

The foregoing description of the two step treatment method of manufacture, and the illustrative discussion of an embodiment is presented to indicate the range of constructions to which the invention applies. The invention and its departure from the prior art being thus disclosed, variations and modifications, including variations in the materials to be used to fabricate polymer samples, vacuum pressures, radiation sources, relative amounts and proportions of pressure, heat and gases applied in the treatment steps and the like, will occur to those having ordinary skill in the art. All such variations and modifications are considered to be within the scope of the invention in which patent rights are asserted, as set forth and defined by the claims appended hereto and their equivalents.

## Claims

**1.** A method of preparing of a prosthesis wear surface component formed of a polymer material, such method comprising the steps of
providing a wear surface blank formed of polymeric material
exposing the blank in a sealed container to a first dose of gamma radiation effective to substantially introduce a desired target level of cross-linking in said polymeric material,
thermally curing the exposed blank in said container above its melt temperature thereby stabilizing said polymer material to form a cross linked blank with enhanced resistance to oxidative degradation over time
forming the blank into a finished component, and
exposing the finished component to a second dose of radiation.

**2.** The method of claim 1, wherein the step of exposing the finished component exposes to a dose of penetrating radiation that is effective to sterilize the component and that is less than said first dose.

**3.** The method of claim 2, wherein the step of exposing the finished component is performed by exposing the component to a dose of gamma radiation sufficient to sterilize the component without generating a chemically significant residual level of free radicals therein.

**4.** The method of claim 1, wherein the step of thermally curing is performed for a combined temperature and duration effective to terminate or link residual free radicals formed by the step of exposing to the first dose of gamma radiation.

**5.** The method of claim 2, wherein the steps of exposing the blank and of exposing the finished component are effected by exposing in a vacuum or inert environment.

**6.** The method of claim 1, wherein the step of thermally curing includes raising to an elevated temperature followed by slowly cooling to ambient.

**7.** The method of claim 1, wherein the step of machining includes cutting a profiled wear surface.

**8.** The method of claim 2, wherein the first dose of gamma radiation and the second dose of radiation form a total dose between 50 and 120 kGy.

**9.** The method of claim 2, wherein the second dose of radiation is below about 35 kGy.

**10.** The method of claim 1, wherein the first dose of gamma radiation is above 50 kGy.

**11.** The method of claim 1, wherein the step of exposing the blank is effected by packaging in a vacuum barrier pouch and exposing in the pouch.

**12.** A prosthesis component formed of a polymeric material and having a wear surface, said component having a near surface region extending to a depth below said wear surface, and characterized in that said near surface region has a degree of cross-linking corresponding to irradiation with a dose of gamma radiation above 50 kGy effective to impart wear resistance for weight-bearing articulation of the prosthesis, and has an oxidation index below 0.15.

**13.** The prosthesis component of claim 12, further having a depth region extending below said near surface region, and an oxidative index less than about 0.10 in said depth region.

**14.** The method of claim 11, wherein the step of exposing the finished component is effected by vacuum foil packaging the finished component and exposing in the package.
high molecular weight polyethylene (UHMWPE) having a depthwise distribution of intra-unit cross linking characteristic of radiation-induced cross linking by radiation of dose D, and a level of residual free radicals characteristic of exposure to a radiation dose < D/2.

**16.** The prosthesis component of claim 12, wherein said component is formed of ultra high molecular weight polyethylene (UHMWPE) and said wear surface is a machined wear surface cut to a depth less than one millimeter.

**17.** A method of forming a prosthesis polymeric wear surface component having enhanced resistance to degradation, such method comprising the steps of
irradiating a blank of bulk or unfinished prosthesis material with a high dose of gamma radiation to introduce a first level of cross-linking corresponding substantially to a desired material strength or hardness
raising the irradiated blank above melt temperature to quench reactive species therein and form a stabilized irradiated blank
machining the stabilized irradiated blank to form a finished component, and
sterilizing and further cross-linking the finished component, to achieve a sterile packaged component having a wear resistant surface of high cross-linking and low reactivity.

**18.** The method of claim 17, further comprising the step of repackaging the blank following the step of machining and prior to the step of sterilizing and further cross-linking.
